Europäisches Patentamt

European Patent Office

Office européen des brevets

(19)

(11) Publication number: **0 213 059**
A2

# EUROPEAN PATENT APPLICATION

(12)

(21) Application number: 86630135.1

(22) Date of filing: 27.08.86

(51) Int. Cl.⁴: **C 12 Q 1/04,** C 12 Q 1/24,
G 01 N 33/569

(30) Priority: 28.08.85 US 770201

(43) Date of publication of application: **04.03.87**
**Bulletin 87/10**

(84) Designated Contracting States: **AT BE CH DE FR GB IT LI NL SE**

(71) Applicant: **RAMOT UNIVERSITY AUTHORITY FOR APPLIED RESEARCH & INDUSTRIAL DEVELOPMENT LTD., Tel Aviv University Ramat Aviv, Tel Aviv (IL)**

(72) Inventor: **Rosenberg, Melvyn, 12 Yeda Am St., Ramat Gan (IL)**
Inventor: **Eli, Ilana, 26 Shazar St., Ramat Gan (IL)**
Inventor: **Weiss, Ervin, 9 Lipsky St., Tel Aviv (IL)**

(74) Representative: **Schmitz, Jean-Marie et al, OFFICE DENNEMEYER S.à.r.l. P.O. Box 1502, L-1015 Luxembourg (LU)**

(54) A method and kit for detecting the presence, extent and location of a specified microorganism on a surface.

(57) A method for detecting the presence, extent and location of a specified microorganism on a surface, particularly caries-associated bacteria on a subject's teeth, comprises contacting the surface (e.g. teeth) with a solid substrate containing at least one substance essential for the growth of the specified microorganism; removing the solid substrate from the surface and immersing it in a liquid growth media lacking the substance essential for the growth of the specified microorganism but otherwise having the necessary growth ingredients for growing the specified microorganism; and incubating the liquid growth medium with the solid substrate immersed therein to promote the growth of the specified microorganism at the interface between the solid substrate and the liquid medium.

EP 0 213 059 A2

A METHOD AND KIT FOR DETECTING THE PRESENCE, EXTENT AND LOCATION OF A SPECIFIED MICROORGANISM ON A SURFACE

The present invention relates to a method for detecting the presence, extent and location of one or more specified microorganism on a surface. The invention is particularly applicable for detecting the presence, extent and location of caries-associated microorganisms on a subject's teeth, and is therefore described below with respect to this application, although it will be appreciated that the invention could advantageously be used in other applications as well.

Until recently, there was little reason to be converned with attempting to localize the development of dental caries, other than from a theoretical viewpoint. Practically speaking, no techniques were available to arrest the development of dental caries, and early information as to their existence was of little practical use.

Recently, however, developments in dental science, such as the use of polymeric sealants, have made it possible to arrest the growth and development of dental caries so that it is now of considerable practical interest to be able to determine and localize the early presence of dental caries in order to arrest their further development.

Traditional examination techniques, such as x-rays and examination with a dental explorer, are capable of detecting the presence of dental caries only at relatively late stages of development when the development can no longer be arrested by the newly devleoped techniques. Furthermore, the use of such techniques on a regular basis is limited because of the health dangers associated with radiation, as well as the discomfort and possible injury to the patient since it has been shown that probing with an explorer damages the dentine in incipient caries. Furthermore, performance of such techniques requires trained personnel and does not allow for detection by the patient himself.

It would, therefore, be very desirable to provide a technique which allows for the early detection and localization of dental caries, which is simple to perform even by untrained personnel, and which is not disturbing to the patient.

The prior art pertinent as background information to the invention of the present application includes the following patents:

Stark U.S. Patent 3,279,068, discloses a method of detecting carious tissue and dental calculus, in which 9-aminoacridine and its hydrochloride are used. However, there is no disclosure of detecting Streptococcus mutans (S. mutans) or other caries-associated microorganisms.

Green U.S. Patent 3,332,743, discloses a diagnositic test for dental caries activity. The test gives a color response at room temperature, based on the sum total of metabolic activity of the saliva. In accordance with that test, a buffered substrate solution containing a suitable indicator dye measures the oxidation rate of a saliva specimen by measuring the time period in which the indicator dye undergoes a distinct color change, which is taken as a measure of dental caries activity. The test does not provide spatial localization of microorganisms associated with the caries-associated activity.

Hoerman et al. U.S. Patent 3,746,624, discloses a 48-hour test for S. mutans in plaque. The method invloves removing a series of specimens from the patient's mouth, generally by insertion of dental floss between the teeth to remove material in the mouth adjacent to the tooth. The floss with the specimen on it is dropped into a test solution. After a 48-hour period, a yellow color indicates the presence of S. mutans. There is no disclosure of detecting the extent and location of caries-associated activity at a specific area of a tooth. Moreover, dental floss, when inserted between two teeth will naturally sample one surface of each tooth at the same time.

Jordan U.S. Patent 3,890,200 describes a selective medium developed for the isolation of S. mutans from human dental plaque.

Stearns at al. U.S. Patent 3,903,252, describes a dental disclosing composition to be used in the mouth. A staining agent associated with the composition adheres to plaque on the teeth. However, the presence of plaque on the teeth does not necessarily indicate the presence of microorganisms which cause caries-associated activity. Thus, dental disclosing compositions, as exemplified by this patent, do not detect the extent and location of caries-associated-activity.

Nakamura et al. U.S. Patent 4,359,455, disclose a diagnostic test composition for dental caries activity. The composition contains a saccharide as a carbon source for promoting the acid-producing capacity of bacteria in the mouth as well as a pH indicator which has a color change point at a pH range of 5 to 7. According to the method, a sampe of dental plaque is collected and introduced into the composition, and then the change of color of the pH indicator contained in the composition is observed with the naked eye. Thus, the composition constitutes an overall measure of bacterial activity in the mouth, and is not intended to determine the location of the activity.

Kashket U.S. Patent 4,368, 272 discloses a method for identifying and locating dental microorganisms including a strip of flexible material having a velvet-like surface to be placed in contact with the selected site in the oral cavity to retain microorganisms on the velvet-like surface, the microorganisms being subsequently transferred onto a substrate, such as agar, for cultivation and identification

Deyloff, U.S. Patent 4,468,456 discloses a medium for differentiating S. mutans. The patent contemplates the innoculation of a culture medium with samples taken from the mouth. There is no disclosure showing the localization of caries-associated activity to specific areas of the teeth.

According to the present invention, there is provided a method for detecting the presence, extent and location of a specified microorganism on a surface, comprising: (a) contacting the surface with a solid substrate containing at least one substance essential for the growth of the specified microorganism; (b) removing the solid substrate from the surface and immersing it in a liquid growth media lacking said at least one substance essential for the growth of the specified microorganism but otherwise having the necessary growth ingredients for growing the specified microorganism; and (c) incubating the liquid growth medium with the solid substrate immersed therein to promote the growth of the specified microorganism at the interface between the solid substrate and the liquid medium.

It will be appreciated that the method can be used for detecting more than one specified microorganism, in which case the mentioned essential substance would be that (or those) for all the microorganisms to be detected. It will also be appreciated that there could be more than one essential substances contained in the solid substrate and lacking in the liquid growth medium.

The invention is particularly applicable for detecting caries-associated microorganisms on a subject's teeth. When so used, the method provides a quick and simple method for detecting the presence, extent and location of the caries-associated microorganism. Since the method uses a liquid rather than a gel as the growth medium, the method can be conveniently performed by non-professional personnel, or by the patient himself, and at no risk or discomfort to the patient.

Known caries-associated bacteria which may be detected in this manner include <u>Streptococcus mutans mitis, anguis, Actinomyces Israeli viscosus,</u> and certain lactobacilli. It is preferrable, particularly when the method is used for detecting caries-associated microorganisms on the teeth of a subject, to rinse the solid substrate with water, such as tap water, before the substrate is immersed in the liquid growth medium, in order to remove non-adherent substances, such as saliva, and non-adherent microorganisms.

The solid substrate so used is preferrably an impressionable material such as chewing gum, to form a solid replica surface. By way of example, a patient may be made to bite on a chewing gum substrate which molds to the configuration of the teeth. The chewing gum itself is also essentially free of antibacterial agents so as to allow the growth of the caries-associated bacteria thereon. In addition to chewing gum, other substrates may be used, for example, plant or vegetable gum, alginate, a paper card, a plaster mold, wax, silicone, cellophane, and the like.

The solid replica substrate contains at least one component which is essential for subsequent bacterial growth, such as a main carbon source, which component is lacking in the liquid growth medium. The carbon source may be, for example, sucrose, galactose, sorbitol, glucose or other sugars, amino acids, sulphates, or alternatively phosphates and ammonium salts. Sucrose has been found particularly effective. After contact between the substrate and the teeth has taken place, the replica substrate is separated from the teeth and washed to remove non-bound cells which might interfere with the localization technique. The replica is then placed into the liquid selective growth medium which contains all of the components necessary for growth of the caries-associated bacteria, except the at least one essential component, e.g., a main carbon source, which is provided within the solid replica. Since the other necessary components for growth are present in the liquid medium, growth occurs primarily at the solid-liquid interface.

Other ingredients necessary for proper growth of the caries-associated bacteria are peptides, amino acids, plant and animal extracts, and phosphate. These substances are present in the liquid growth medium.

In addition to containing growth agents, the liquid growth medium, and/or substrate, may additionally contain components which inhibit the growth of non-caries-associated bacteria. Such materials include, by way of example, potassium tellurite, trypan blue, gentian violet, etc. A preferred material which may be included is bacitracin to increase selectivity for S. mutans. Likewise the pH of the liquid may also be adjusted to a sufficiently low level as an alternative or cumulative means of inhibiting growth of non-caries-associated bacteria.

One or more indicator substances are further included, preferably in the liquid growth medium, which are absorbed by, or otherwise associated with, the microorganism during the incubation of the substrate in the liquid growth medium. By way of example, such an indicator may include gentian violet. This indicator allows for visualization, and thereby facilitates identification of the caries-associated bacteria. Gentian violet serves not only as an indicator, but also as an inhibitor of non-caries-associated bacteria

Incubation of the solid replica substrate within the liquid medium occurs at temperatures of approximately 35-37°C for periods of approximately 6-24 hours sufficient to allow for significant bacterial growth to permit visualization.

Following incubation of the solid replica within the liquid growth medium, bacterial growth can be easily perceived by absorption of the indicator at certain locations on the replica substrate. This in turn can be correlated with the caries-associated activity of the tooth surface.

Another indication technique which may be used includes the use of standard immunological reagents which make visualization possible. Such techniques may, for example, include incubating the substrate in an immune serum which includes fluorescent

0213059

- 8 -

or enzyme-containing antibodies which specifically bind with caries-associated bacteria during incubation of the solid replica substrate.

### EXAMPLE

The method will now be exemplified by way of the following non-limiting example:

The patient is asked to bite down on chewing gum serving as a solid replica substrate such that a visual imprint of the tooth surface on the chewing gum is made. The gum has the following composition: 20% Paloya gum base (L.A. Dreyfus Co.), 1% glycerol, 60% sucrose, and 19% glucose. The chewing gum may be exposed on only one of its surfaces to the teeth, or may be exposed on both surfaces to the upper and lower teeth. Also, the buccal and lingual sides of the teeth may be exposed.

The chewing gum is then carefully removed from the mouth of the patient, washed briefly in tap water, and immersed in a liquid medium having the following composition: 10 g Bactotryptose, 10 g proteose peptone, 75 mg. trypan blue, 0.8 mg gentian violet 10 ug potassium tellurite, 500 units bacitracin, and water to make one liter The chewing gum is permitted to incubate within the liquid medium for approximately 6-24 hours at a temperature of approximately 35  2 degrees Centigrade. After incubation, the liquid medium is drained, and the substrate is visually examined for evidence of caries-associatedly-related microorganisms, which organisms have absorbed the visually-observable dye or dyes.

The patient, technician or dentist is then able to identify the presence, extent and location of the caries-associated bacteria by correlating the replica with the teeth of the patient.

Thus, although previous techniques have allowed for procedures which merely test for the presence of caries-associated activity, such techniques have not allowed for the detection also of the extent and location of the such bacteria and have thus have given little more than evidence that a cavity is about to occur. This information would then require the more careful routine examinations which have been traditionally performed and which suffer from the drawbacks mentioned above. Using the technique of the invention, the location and extent of the caries-associated bacteria is detected, and therefore the bacteria may be treated at an early stage using procedures which are now becoming available. Also, the technique involves inexpensive materials which (unlike x-rays) are harmless, do not require peripheral equipment other than an incubator, and (being a liquid) are very simple to handle by unskilled persons, as compared to gels.

Although the invention has been described with reference to a particular application and materials, it is to be understood that the invention is not limited to the particulars described. For example, although the invention has been described with reference to chewing gum as the solid replica substrate, it is clear that other impressionable materials, such as cardboard sheets, alginates, plaster molds, and the like may be used. Likewise, although the invention has been described with respect to a particular nutrient source in the substrate, it is clear that other nutrient growth sources may be present in either the substrate or the liquid medium to achieve the desired objectives. Finally, although described with reference specifically to tooth examination, it is clear that the localization concept of the invention is not limited to teeth and may be used whereever one desires to determine the presence, extent and location of one or more suspected bacteria or other microorganisms on a surface.

## CLAIMS

1. A method for detecting the presence, extent and location of a specified microorganism on a surface, comprising:

(a) contacting the surface with a solid substrate containing at least one substance essential for the growth of the specified microorganism;

(b) removing said solid substrate from the surface and immersing it in a liquid growth media lacking said at least one substance essential for the growth of the specified microorganism but otherwise having the necessary growth ingredients for growing the specified microorganism; and

(c) incubating said liquid growth medium with the solid substrate immersed therein to promote the growth of the specified microorganism at the interface between the solid substrate and the liquid medium.

2. The method according to Claim 1, wherein: said surface is a subject's teeth, the solid substrate is an impressionable material applied to the subject's teeth, and said specified microorganism is caries-associated bacteria.

3. The method according to Claim 2, wherein said impressionable material is chewing gum.

4. The method according to any one of Claims 1-3, including the further step of rinsing the solid substrate with water before immersing it in the liquid growth medium in order to remove non-adherent substances and microorganisms.

5.. The method according to any one of Claims 1-4, wherein said at least one substance essential for the growth of the specified microorganism contained in the solid substrate, but lacking in said liquid growth medium, is a carbon source.

6. The method according to Claim 5, wherein said carbon source is sucrose.

7. The method according to any one of Claims 1-6, wherein said liquid growth medium includes at least one indicator substance which becomes associated with the microorganism during the incubation of the substrate in the liquid growth medium, to facilitate identification of the specified microorganism.

8. The method according to Claim 7, wherein said at least one indicator substance in the liquid growth medium is a chromogenic selective agent absorbed by the microorganism during the incubation of the substrate in the liquid growth medium, to facilitate visual observation.

9. The method according to Claim 7, wherein said at least one indicator substance is an immune serum containing fluorescent or enzyme-containing antibodies associated with the specified microorganism during incubation of the substrate in the liquid growth medium.

10. The method according to any one of Claims 1-9, wherein said solid substrate and/or said liquid growth medium further includes a substance inhibiting the growth of microorganisms except the specified microorganism.

11. A kit for use in detecting the presence, extent and location of a specified microorganism on a surface, comprising: a solid substrate containing at least one substance essential for the growth of the specified microorganism; and a liquid growth medium lacking said at least one substance essential for the growth of the specified microorganism but otherwise having the necessary growth ingredients for growing the specified microorganism, such that by contacting the said surface with the solid substrate, immersing the solid substrate in the liquid growth medium, and then incubating said liquid growth medium with the solid substrate immersed therein, the growth of the specified microorganism is promoted at the interface between the solid substrate and the liquid growth medium.